(19) Europäisches Patentamt
European Patent Office
Office européen des brevets

(11) EP 0 980 368 B1

(12) FASCICULE DE BREVET EUROPEEN

(45) Date de publication et mention de la délivrance du brevet:
**22.10.2003 Bulletin 2003/43**

(21) Numéro de dépôt: **98924365.4**

(22) Date de dépôt: **04.05.1998**

(51) Int Cl.[7]: **C07D 413/04**, C07D 413/14, A61K 31/41, A61K 31/445

(86) Numéro de dépôt international:
**PCT/FR98/00887**

(87) Numéro de publication internationale:
**WO 98/050381 (12.11.1998 Gazette 1998/45)**

# (54) DERIVES DE 3-(PYRROLIDIN-3-YL)-1,3,4-OXADIAZOL-2(3H)-ONE ET LEUR UTILISATION COMME LIGANDS 5-HT4

3-(3-PYRROLIDINYL)-2(3H)-1,3,4-OXADIAZOLONDERIVATE UND IHRE VERWENDUNG ALS 5-HT4 LIGANDEN

3-(PYRROLIDIN-3-YL)-1,3,4-OXADIAZOL-2(3H)-ONE DERIVATIVES AND THEIR USE AS 5-HT4 LIGANDS

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **06.05.1997 FR 9705539**

(43) Date de publication de la demande:
**23.02.2000 Bulletin 2000/08**

(73) Titulaire: **SANOFI-SYNTHELABO**
**75013 Paris (FR)**

(72) Inventeurs:
- **LOCHEAD, Alistair**
  **F-94220 Charenton (FR)**
- **JEGHAM, Samir**
  **F-95100 Argenteuil (FR)**
- **JEUNESSE, Jean**
  **F-94500 Champigny sur Marne (FR)**
- **NEDELEC, Alain**
  **F-92700 Colombes (FR)**

(74) Mandataire: **Ludwig, Jacques et al**
**Sanofi-Synthélabo**
**Service Brevets**
**174, avenue de France**
**75013 Paris (FR)**

(56) Documents cités:
**WO-A-93/16072**
**WO-A-95/32965**
**WO-A-97/17345**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des composés de formule générale (I)

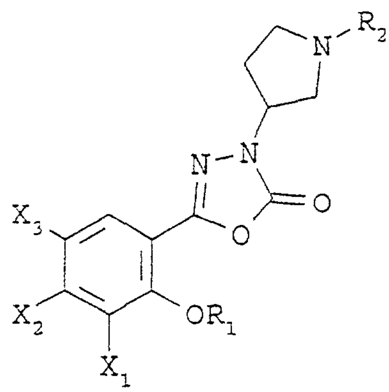

(I)

dans laquelle
$R_1$ représente un atome d'hydrogène ou un groupe $(C_1-C_4)$alkyle ou $(C_3-C_7)$ cycloalkylméthyle,
$X_1$ représente un atome d'hydrogène ou un groupe $(C_1-C_4)$alcoxy ou bien
$OR_1$ et $X_1$ représentent ensemble un groupe de formule $-OCH_2O-$, $-O(CH_2)_2-$, $-O(CH_2)_3-$, $-O(CH_2)_2O-$ ou $-O(CH_2)_3O-$,
$X_2$ représente un atome d'hydrogène, un groupe amino ou un groupe de formule générale $-NHCO_2R$ dans laquelle R représente un groupe $(C_1-C_4)$alkyle ou phényl$(C_1-C_2)$alkyle,
$X_3$ représente un atome d'hydrogène ou d'halogène, et
$R_2$ représente un atome d'hydrogène ou un groupe $(C_1-C_6)$alkyle, phényl $(C_1-C_4)$ alkyle ou [(4-diméthylamino)pipéridin-1-ylcarbonyl] $(C_2-C_4)$alkyle.

L'atome de carbone par lequel le cycle pyrrolidine est lié au reste de la molécule écant asymétrique, les composés de l'invention peuvent exister à l'état d'énantiomères purs ou de mélanges d'énantiomères.

Ils peuvent également exister à l'état de bases ou de sels d'additions à des acides.

Les composés de formule générale (I) peuvent être préparés selon un procédé illustré par le schéma qui suit.

Schéma

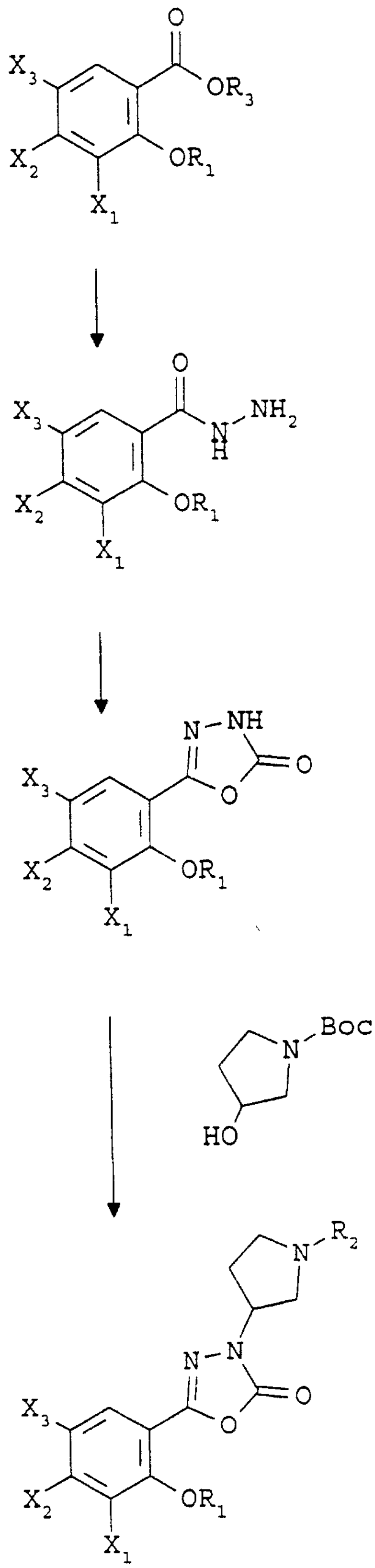

(II)

(III)

(IV)

(V)

(I)

On fait réagir un ester de formule générale (II), dans laquelle $R_1$, $X_1$, $X_2$ et $X_3$ sont tels que définis ci-dessus et $R_3$ représente un groupe méthyle ou éthyle, avec l'hydrate d'hydrazine, en l'absence de solvant ou dans un solvant polaire protique, par exemple l'éthanol, pour obtenir un hydrazide de formule générale (III) dont on obtient la cyclisation en oxadiazole de formule générale (IV) soit au moyen de phosgène, dans un solvant aprotique, par exemple le dioxane, soit au moyen de chloroformiate de phényle, dans un solvant aprotique, par exemple le toluène.
Lorsque, dans la formule générale (II), $X_2$ représente un groupe amino, ce dernier réagit avec le phosgène, et on estérifie le produit obtenu avec un alcool de formule générale ROH, dans laquelle R est défini comme ci-dessus, le groupe amino étant ainsi protégé par un groupe $-CO_2R$.
On fait ensuite réagir l'oxadiazole de formule générale (IV) avec un alcool de formule générale (V), dans laquelle Boc représente un groupe (1,1-diméthyléthoxy)carbonyle, en présence de triphénylphosphine et d'azodicarboxylate d'éthyle, dans un solvant aprotique, par exemple le tétrahydrofurane, puis on élimine le groupe protecteur (1,1-diméthyléthoxy)carbonyle au moyen d'acide trifluoroacétique.
On obtient ainsi un composé de formule générale (I) dans laquelle $R_2$ représente un atome d'hydrogène.
On peut ensuite, si on le désire, soumettre ce dernier à une alkylation au moyen d'un dérivé de formule générale $R_2$-X, dans laquelle X représente un groupe partant, par exemple un atome d'halogène ou un groupe méthanesulfonate ou paratoluènesulfonate, et $R_2$ est tel que défini ci-dessus, mais différent d'un atome d'hydrogène, en présence de triéthylamine, dans un solvant aprotique, par exemple l'acétonitrile. Finalement, lorsque $X_2$ est un groupe $-NHCO_2R$, on peut, si on le désire, éliminer le groupe protecteur en milieu acide.

Les esters de départ de formule générale (II) et/ou les acides correspondants sont décrits notamment dans les demandes de brevets EP-0231139, EP-0234872, WO-8403281, WO-9316072 et WO-9419344.

Les alcools de formule générale (V) sont connus ou peuvent être préparés selon toutes méthodes connues ; le (*R*/*S*)-1-[(1,1-diméthyléthoxy)carbonyl]pyrrolidin-3-ol est décrit dans *J. Am. Chem. Soc.* (1982) **104** 5852-5853, et l'énantiomère (*R*) est décrit dans *Tetrahedron* (1988) **44**(17) 5479-5486.

Les exemples suivants illustrent en détail la préparation de quelques composés selon l'invention. Les microanalyses élémentaires et les spectres IR et RMN confirment les structures des composés obtenus. Les numéros des composés indiqués entre parenthèses dans les titres correspondent à ceux du tableau donné plus loin. Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

<u>Exemple 1</u> (Composé N°9).

[2-Chloro-4-(5-oxo-4-(pyrrolidin-3-yl)-4,5-dihydro-1,3,4-oxadiazol-2-yl)-5-méthoxyphényl]carbamate de phénylméthyle.

1.1. Hydrazide de l'acide 4-amino-5-chloro-2-méthoxyben_ zoïque.

Dans un réacteur de 1 l on introduit 51,5 g (0,239 mole) de 4-amino-5-chloro-2-méthoxybenzoate de méthyle en suspension dans 460 ml d'éthanol. On ajoute, en 15 min, 119 g (2,39 moles) d'hydrate d'hydrazine et on chauffe le mélange au reflux pendant 15h.
On refroidit le mélange à l'aide d'un bain de glace, on collecte le précipité par filtration, on le rince à l'éthanol et on le sèche sous pression réduite à 80°C pendant 2h30. On obtient ainsi 47,5 g de produit.
Point de fusion : 211°C.

1.2. [2-Chloro-5-méthoxy-4-(5-oxo-4,5-dihydro-1,3,4-oxadiazol-2-yl)phényl]carbamate de phénylméthyle.

Dans un réacteur de 3 l on ajoute, goutte à goutte, en l'espace d'une heure, à température ambiante et sous agitation magnétique, 461 ml (0,875 mole) d'une solution de phosgène 1,93M dans le toluène, à une suspension de 37,7 g (0,175 mole) d'hydrazide de l'acide 4-amino-5-chloro-2-méthoxybenzoïque dans 1200 ml de dioxanne.
On agite le mélange à température ambiante pendant une nuit, puis on le chauffe à 80°C pendant 1h. On chasse l'excès de phosgène par passage d'un courant d'argon à cette température pendant 2h. On ajoute alors 72 ml (0,7 mole) d'alcool benzylique et on continue à chauffer pendant 1h à 100°C. On refroidit, on concentre sous pression réduite et on triture le résidu dans l'éther isopropylique. On filtre et on sèche le solide obtenu. On obtient ainsi 60,3 g de produit.
Point de fusion : 214°C.

1.3. [2-Chloro-4-[3-[1-(1,1-diméthyléthoxy)carbonyl]pyrro_ lidin-3-yl]-5-oxo-4,5-dihydro-1,3,4-oxadiazol-2-yl]-5-méthoxyphényl]carbamate de phénylméthyle.

Dans un ballon tricol de 500 ml on introduit 9,8 g (26,1 mmoles) de [2-chloro-5-méthoxy-4-(5-oxo-4,5-dihydro-1,3,4-oxadiazol-2-yl)phényl]carbamate de phénylméthyle en solution dans 100 ml de tétrahydrofurane, 11,6 g (44,3 mmoles) de triphénylphosphine et 4,9 g (26,1 mmoles) de 1-[(1,1-dimé_ thyléthoxy)carbonyl]pyrrolidin-3-ol. Tout en agitant le mélange à 0°C, on ajoute, goutte à goutte, une solution de 5,9 g (33,9 mmoles) d'azodicarboxylate d'éthyle dans 6 ml de tétrahydrofurane et on poursuit l'agitation à température ambiante pendant 24 h.
On évapore le solvant sous pression réduite et on purifie le résidu par chromatographie sur gel de silice en éluant avec des mélanges 65/35, 60/40 et finalement 30/70 d'heptane et d'acétate d'éthyle. On obtient ainsi 10,1 g de produit.
Point de fusion : 138°C.

1.4. [2-Chloro-4-(5-oxo-4-(pyrrolidin-3-yl)-4,5-dihydro-1,3,4-oxadiazol-2-yl)-5-méthoxyphényl]carbamate de phénylméthyle.

Dans un ballon tricol de 500 ml on introduit 10,1 g (18,5 mmoles) de [2-chloro-4-[3-[1-(1,1-diméthyléthoxy) carbo_ nyl]pyrrolidin-3-yl]-5-oxo-4,5-dihydro-1,3,4-oxadiazol-2-yl]-5-méthoxyphényl]carbamate de phénylméthyle en solution dans 100 ml de dichlorométhane. On refroidit la solution à 0°C, on ajoute, goutte à goutte, 14,3 ml (185 mmoles) d'acide triflu_ oroacétique et on agite le mélange à température ambiante pendant 17h.
On évapore le solvant sous pression réduite on dissout le résidu dans 1500 ml d'éthanol et on l'acidifie par addition de 4,5 ml d'une solution d'acide chlorhydrique dans l'éthanol. On obtient un solide blanc que l'on reprend dans 80 ml d'eau et 80 ml de chloroforme, on ajoute de l'ammo_ niaque et on extrait au chloroforme. On évapore le solvant sous pression réduite pour obtenir 4,7 g de produit sous forme de solide blanc.
Point de fusion : 131°C.

Exemple 2 (Composé N°10).

[2-Chloro-5-méthoxy-4-[5-oxo-4-(1-butylpyrrolidin-3-yl)-4,5-dihydro-1,3,4-oxadiazol-2-yl]phényl]carbamate de phénylméthyle.

Dans un ballon tricol de 100 ml on place 1,5 g (3,37 mmoles) de [2-chloro-4-(5-oxo-4-(pyrrolidin-3-yl)-4,5-dihydro-1,3,4-oxadiazol-2-yl)-5-méthoxyphényl]carbamate de phénylméthyle en solution dans 26 ml d'acétonitrile et 1,41 ml (10,1 mmoles) de triéthylamine. On ajoute une solution de 0,6 g (4,38 mmoles) de 1-bromobutane dans 4m l d'acétonitrile et on chauffe à 60°C pendant 15 h.
On évapore le solvant sous pression réduite et on purifie le résidu par chromatographie sur gel de silice en éluant avec un mélange 99/1/0,1, 98/2/0,2 et finalement 95/5/0,5 de chloroforme, méthanol et ammoniaque. On obtient ainsi 1,35 g de produit sous forme de solide blanc.
Point de fusion : 114°C.

Exemple 3 (Composé N°8).

Bromhydrate de 5-(4-amino-5-chloro-2-méthoxyphényl)-3-[3-(1-butylpyrrolidin-3-yl)]-1,3,4-oxadiazol-2(3*H*)-one.

Dans un ballon tricol de 50 ml on introduit 1,29 g (2,58 mmoles) de [2-chloro-5-méthoxy-4-[5-oxo-4-(1-butylpyrrolidin-3-yl) -4,5-dihydro-1,3,4-oxadiazol-2-yl]phényl]carbamate de phénylméthyle en solution dans 13 ml d'acide acétique, on ajoute 3,2 ml d'une solution d'acide bromhydrique dans l'acide acétique à 33% et on agite le mélange à température ambiante pendant 46 h.
On évapore le solvant sous pression réduite et on lave le solide obtenu à l'éther et puis à l'éthanol et on le recristallise dans l'éthanol pour obtenir 0,53 g de produit pur sous forme de solide blanc.
Point de fusion : 197°C.

Exemple 4 (Composé N°11).

(S)-[6-Chloro-8-[(4-pyrrolidin-3-yl)-5-oxo-4,5-dihydro-1,3,4-oxadiazol-2-yl]-2,3-dihydro-1,4-benzodioxin-5-yl)carbamate de phénylméthyle.

4.1. 8-Amino-2,3-dihydro-1,4-benzodioxine-5-carboxylate d'éthyle.

Dans un ballon tricol de 2 l contenant 772 ml d'éthanol refroidi à -40°C, sous agitation, on introduit lentement 23,5 g (0,198 mole) de chlorure de thionyle, on maintient l'agitation à cette température pendant 1h, on ajoute lentement, en 15 min, 38,6 g (0,198 mole) d'acide 8-amino-2,3-dihydro-1,4-benzodioxine-5-carboxylique en solution dans 100 ml d'éthanol, et on laisse le mélange revenir à température ambiante pendant une nuit.
On le chauffe au reflux pendant 4h, on évapore le solvant sous pression réduite, on reprend le résidu avec de l'eau et du carbonate de sodium et on l'extrait avec du chloroforme. Après lavage, séchage et évaporation de la phase organique on obtient 34,06 g d'ester sous forme de solide blanc.
Point de fusion : 112°C.

4.2. 8-Amino-7-chloro-2,3-dihydro-1,4-benzodioxine-5-car_ boxylate d'éthyle.

Dans un ballon de 1 l on introduit 37 g (0,165 mole) de 8-amino-2,3-dihydro-1,4-benzodioxine-5-carboxylate d'éthyle en solution dans 370 ml de dioxane, on ajoute, à température ambiante et sous agitation magnétique, 23,2 g (0,174 mole) de *N*-chlorosuccinimide et on agite le mélange pendant une nuit. On le dilue à l'eau, on l'extrait à l'acétate d'éthyle et, après traitement habituel de la phase organique, on obtient 42 g de composé qu'on recristallise dans un mélange d'éther diéthylique et d'éther diisopropylique.
Point de fusion : 105-106°C.

4.3. Hydrazide de l'acide 8-amino-7-chloro-2,3-dihydro-1,4-benzodioxine-5-carboxylique.

Dans un réacteur de 1 l on introduit 38,4 g (0,149 mole) de 8-amino-7-chloro-2,3-dihydro-1,4-benzodioxine-5-carboxylate d'éthyle en suspension dans 150 ml d'éthanol, on ajoute, en 15 min, 149 g (2,98 mole) d'hydrate d'hydrazine et on chauffe le mélange au reflux pendant 1h.
On le refroidit à l'aide d'un bain de glace, on recueille le précipité par filtration, on le lave à l'éthanol et on le sèche sous pression réduite.
On obtient 33 g de composé.
Point de fusion : 227-231°C.

4.4. [6-Chloro-8-(5-oxo-4,5-dihydro-1,3,4-oxadiazol-2-yl)-2,3-dihydro-1,4-benzodioxin-5-yl]carbamate de phényl_ méthyle.

Dans un réacteur de 1 l, à température ambiante et sous agitation magnétique, on introduit 32,6 g d'hydrazide de l'acide 8-amino-7-chloro-2,3-dihydro-1,4-benzodioxine-5-carboxylique et 330 ml de dioxane, on ajoute à cette suspension, goutte à goutte, et en l'espace d'une heure et demie, 310 ml (0,4 mole) d'une solution de phosgène 0,193M dans le toluène, on agite le mélange à température ambiante pendant une nuit et on le chauffe au reflux pendant 5h. On chasse l'excès de phosgène à cette température par passage d'un courant d'argon pendant 2h, on refroidit le mélange, on le concentre sous pression réduite, on reprend le résidu avec 200 ml d'alcool benzylique, on le chauffe à 100°C pendant une nuit, on refroidit le mélange, on le concentre sous pression réduite et on triture le résidu dans l'éther diisopropylique. Après filtration et séchage on obtient 52,6 g de composé.
Point de fusion : 230°C.

4.5. (*S*)-[6-Chloro-8-[4-[1-[(1,1-diméthyléthoxy)carbonyl]_ pyrrolidin-3-yl]-5-oxo-4,5-dihydro-1,3,4-oxadiazol-2-yl]-2,3-dihydro-1,4-benzodioxin-5-yl]carbamate de phénylméthyle.

Dans un ballon tricol de 500 ml on place 10,8 g (26,7 mmoles) de [6-chloro-8-(5-oxo-4,5-dihydro-1,3,4-oxadiazol-2-yl)-2,3-dihydro-1,4-benzodioxin-5-yl]carbamate de phénylméthyle en solution dans 110 ml de tétrahydrofurane et 11,9 g (45,4 mmoles) de triphénylphosphine. On ajoute une solution de 5,0 g (26,7 mmoles) de (*R*)-1-[(1,1-diméthyléthoxy)carbonyl]pyrrolidin-3-ol dans 50 ml de tétrahydrofurane et on refroidit la solution à 0°C. On ajoute ensuite, goutte à goutte, une solution de 5,5 ml (34,7 mmoles) d'azodicarboxylate d'éthyle dans 6 ml de tétrahydrofurane et on poursuit l'agitation pendant 20 h.
On évapore le solvant sous pression réduite et on purifie le résidu par chromatographie sur gel de silice en éluant avec

un mélange 99/1 de chloroforme et de méthanol. On récupère des fractions contenant le produit et on les repurifie par chromatographie sur gel de silice en éluant avec du chloroforme. On obtient ainsi 4,8 g de produit pur sous forme de solide blanc.
Point de fusion : 157°C $[\alpha]_D^{20}$ = +36,8° (c=1,$CHCl_3$).

4.6. (S)-[6-Chloro-8-[(4-pyrrolidin-3-yl)-5-oxo-4,5-dihydro-1,3,4-oxadiazol-2-yl]-2,3-dihydro-1,4-benzodioxin-5-yl)carbamate de phénylméthyle.

Dans un ballon tricol de 250 ml on introduit 4,7 g (8,2 mmoles) de (*S*)-[6-chloro-8-[4-[1-[(1,1-diméthyléthoxy) carbonyl]pyrrolidin-3-yl]-5-oxo-4,5-dihydro-1,3,4-oxadiazol-2-yl]-2,3-dihydro-1,4-benzodioxin-5-yl)carbamate de phénylméthyle en solution dans 47 ml de dichlorométhane. On refroidit la solution à 0°C, on ajoute 6,3 ml (82,0 mmoles) d'acide trifluoroacétique et on agite à température ambiante pendant 4h.
On évapore le solvant sous pression réduite et on ajoute 40 ml d'eau et 40 ml de chloroforme. On alcalinise la solution par addition de 4 ml d'ammoniaque concentré, on extrait au dichlorométhane et on évapore la phase organique sous pression réduite. On purifie le résidu par chromatographie sur gel de silice en éluant avec un mélange 95/5/0,5 de chloroforme, méthanol et ammoniaque pour obtenir 3,5g de produit sous forme de solide blanc.
Point de fusion : 126°C $[\alpha]_D^{20}$ = -3,0° (c=1,$CHCl_3$).

<u>Exemple 5</u> (Composé N°3).

(*S*)-5-(8-Amino-7-chloro-2,3-dihydro-1,4-benzodioxin-5-yl)-3-[1-[5-[4-(diméthylamino)pipéridin-1-yl]-5-oxo_ pentyl] pyrrolidin-3-yl]-1,3,4-oxadiazol-2-(3*H*)-one.

5.1. (*S*)-3-[5-[7-Chloro-8-[[(phénylméthoxy)carbonyl]amino]-2,3-dihydro-1,4-benzodioxin-5-yl]2-oxo-2,3-dihydro-1,3,4-oxadiazol-3-yl]pyrrolidine-1-pentanoate de méthyle.

Dans un ballon tricol de 250 ml on introduit 3,46 g (7,32 mmoles) de (*S*)-[6-chloro-8-[(4-pyrrolidin-3-yl)-5-oxo-4,5-dihydro-1,3,4-oxadiazol-2-yl]-2,3-dihydro-1,4-benzodioxin-5-yl)carbamate de phénylméthyle en solution dans 62 ml d'acéto_ nitrile et 6,2 g (44 mmoles) de triéthylamine. On ajoute, à température ambiante, 2,74 g (19 mmoles) de 5-chloropentan_ oate de méthyle en solution dans 7 ml d'acétonitrile et on chauffe le milieu réactionnel à reflux pendant 120 h.
On évapore le solvant sous pression réduite, on reprend le résidu dans 70 ml d'eau et 70 ml de chloroforme, on sépare la phase organique, on évapore le solvant sous pression réduite et on purifie le résidu liquide par chromatographie sur gel de silice en éluant avec un mélange 98/2/0,2 de chloroforme, méthanol et ammoniaque. On obtient ainsi 0,54 g de produit sous forme de pâte collante.
$[\alpha]_D^{20}$ = +29,2° (c=1,$CHCl_3$).

5.2. Acide (*S*)-3-[5-[8-amino-7-chloro-2,3-dihydro-1,4-benzo_ dioxin-5-yl]2-oxo-2,3-dihydro-1,3,4-oxadiazol-3-yl]pyr_ rolidine-1-pentanoïque.

Dans un ballon de 25 ml on place 0,50 g (0,85 mmole) de (*S*)-3-[5-[7-chloro-8-[[(phénylméthoxy)carbonyl] amino]-2,3-dihydro-1,4-benzodioxin-5-yl]2-oxo-2,3-dihydro-1,3,4-oxadia zol-3-yl]pyrrolidine-1-pentanoate de méthyle en solution dans 5 ml d'acide chlorhydrique aqueux concentré et on chauffe le milieu réactionnel à reflux pendant 22 h. On évapore l'acide chlorhydrique aqueux sous pression réduite pour obtenir 0,25 g de produit brut qu'on utilise tel quel dans l'étape suivante.

5.3. (*S*)-5-(8-Amino-7-chloro-2,3-dihydro-1,4-benzodioxin-5-yl)-3-[1-[5-[4-(diméthylamino)pipéridin-1-yl]-5-oxo_ pentyl]pyrrolidin-3-yl]-1,3,4-oxadiazol-2-(3*H*)-one.

Dans un ballon tricol de 25 ml on introduit 0,24 g (0,51 mmole) d'acide (*S*)-3-[5-[7-chloro-8-amino-2,3-dihydro-1,4-benzodioxin-5-yl]2-oxo-2,3-dihydro-1,3,4-oxadiazol-3-yl]pyrrolidine-1-pentanoïque en solution dans 2,5 ml de diméthyl_ formamide et 0,14 ml (1,01 mmoles) de triéthylamine. On ajoute, goutte à goutte, une solution de 0,16 g (1,01 mmole) de 1,1'-carbonylbis-1*H*-imidazole dans 1,1 ml de diméthylformamide et on agite à température ambiante pendant 2 h 30 min. On ajoute une solution de 0,76 mmole de 4-(diméthylamino)_ pipéridine dans 1,8 ml de diméthylformamide (préparée préalablement par chauffage d'une suspension de 0,16 g (0,76 mmole) du dichlorhydrate de 4-(diméthylamino)pipéridine dans 1,8 ml de diméthylformamide et 0,28 ml (2,02 mmoles) de triéthyl_ amine à 60°C pendant 2 h) et on agite à température ambiante pendant 18 h.
On verse le milieu réactionnel dans 30 ml d'eau et on extrait au chloroforme. On purifie le produit par chromatographie

sur gel de silice en éluant avec un mélange 95/5/0,5 puis 80/20/2 de chloroforme, méthanol et ammoniaque. On obtient ainsi 0,08 g de produit sous forme de pâte collante.
$[\alpha]_D^{20}$ = +26° (c=0,4,$CHCl_3$).

Le tableau qui suit illustre les structures chimiques et les propriétés physiques de quelques composés de l'invention.

Tableau

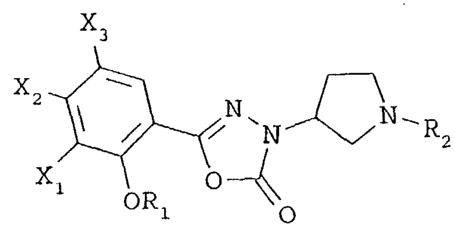

(I)

| N° | $OR_1$ | $X_1$ | $X_2$ | $X_3$ | $R_2$ | Conf. | Sel | F (°C) | $[\alpha]_D^{20}$ |
|---|---|---|---|---|---|---|---|---|---|
| 1 | $-O(CH_2)_2O-$ | | $NH_2$ | Cl | $-(CH_2)_2C_6H_5$ | *R/S* | HCl | 219 | - |
| 2 | $-O(CH_2)_2O-$ | | $NH_2$ | Cl | $(CH_2)_4CONC_5H_9N(CH_3)_2$ | *R/S* | HCl | 148-150 | - |
| 3 | $-O(CH_2)_2O-$ | | $NH_2$ | Cl | $(CH_2)_4CONC_5H_9N(CH_3)_2$ | *S* | - | pâte | +26 (c=0,4, $CH_2Cl_2$) |
| 4 | $-O(CH_2)_2O-$ | | $NH_2$ | Cl | $-(CH_2)_3CH_3$ | *R/S* | HBr | 218-221 | - |
| 5 | $-O(CH_2)_2O-$ | | $NH_2$ | Cl | $-CH(CH_3)_2$ | *R/S* | HBr | 250 | - |
| 6 | $-O(CH_2)_2O-$ | | $NH_2$ | Cl | $(CH_2)_3CONC_5H_9N(CH_3)_2$ | *R/S* | 2ox. | 145-150 | - |
| 7 | $OCH_3$ | H | $NH_2$ | Cl | $-(CH_2)_2C_6H_5$ | *R/S* | HBr | 200-204 | - |
| 8 | $OCH_3$ | H | $NH_2$ | Cl | $-(CH_2)_3CH_3$ | *R/S* | HBr | 197 | - |
| 9 | $OCH_3$ | H | $NHCO_2CH_2C_6H_5$ | Cl | H | *R/S* | - | 131 | - |
| 10 | $OCH_3$ | H | $NHCO_2CH_2C_6H_5$ | Cl | $-(CH_2)_3CH_3$ | *R/S* | - | 114 | - |
| 11 | $-O(CH_2)_2O-$ | | $NHCO_2CH_2C_6H_5$ | Cl | H | *S* | - | 126 | -3,0 (c=1, $CHCl_3$) |

Dans la colonne "R", "$NC_5H_9N(CH_3)_2$" désigne un groupe 4-(diméthylamino)pipéridin-1-yle.

Dans la colonne "Sel", "-"désigne un composé à l'état de base, "HCl" désigne un chlorhydrate, "HBr" désigne un bromhydrate et "2ox." désigne un dioxalate.

Les composés de l'invention ont fait l'objet d'essais qui ont mis en évidence leur intérêt comme substances à activités thérapeutiques.

Ainsi les composés de l'invention ont été étudiés quant à leur affinité vis-à-vis des récepteurs 5-$HT_4$ dans le striatum de cobaye selon la méthode décrite par Grossman et coll. dans *Br. J. Pharmacol*. (1993) **109** 618-624.

On euthanasie des cobayes (Hartley, Charles River, France) de 300 à 400 g, on prélève les cerveaux, on excise les striata et on les congèle à -80°C.

Le jour de l'expérience on décongèle le tissu à +4°C dans 33 volumes de tampon HEPES-NaOH (50 mM, pH = 7,4 à 20°C), on l'homogénéise à l'aide d'un broyeur Polytron™, on centrifuge l'homogénat à 48000 g pendant 10 min, on récupère le culot, on le remet en suspension, on le centrifuge de nouveau dans les mêmes conditions et on remet le culot final en suspension dans du tampon HEPES-NaOH, à raison de 30 mg de tissu par ml. On fait incuber 100 µl de cette suspension membranaire à 0°C pendant 120 min en présence de [$^3$H]GR113808 (ligand décrit dans l'article cité, activité spécifique 80-85 Ci/mmole) dans un volume final de 1 ml de tampon HEPES-NaOH (50 mM, pH = 7,4), en présence ou en absence de composé à tester. On arrête l'incubation par filtration sur filtre Whatman GF/B préalablement traité avec de la polyéthylèneimine à 0,1%, on rince chaque tube avec 4 ml de tampon à 0°C, on filtre de nouveau et on mesure la radioactivité retenue sur le filtre par scintigraphie liquide.

On détermine la liaison non spécifique en présence de sérotonine 30 µM. La liaison spécifique représente 90% de la radioactivité totale récupérée sur le filtre.

Pour chaque concentration de composé étudié on détermine le pourcentage d'inhibition de la liaison spécifique du [$^3$H] GR113808 puis la $CI_{50}$, concentration du composé testé qui inhibe 50% de la liaison spécifique.

Les $CI_{50}$ des composés les plus actifs se situent entre 0,7 et 15 nM.

Les composés de l'invention ont aussi été étudiés quant à leurs effets agonistes ou antagonistes vis-à-vis des récepteurs 5-$HT_4$ dans l'oesophage de rat selon la méthode décrite par Baxter et coll. dans *Naunyn Schmied. Arch. Pharmacol*. (1991) **343** 439.

On utilise des rats mâles Sprague-Dawley pesant de 300 à 450 g. On prélève rapidement un fragment d'environ 1,5 cm de la partie terminale de l'oesophage, on élimine la couche musculaire, on ouvre longitudinalement la tunique muqueuse musculaire interne, on la monte dans une cuve à organe isolé contenant une solution de Krebs-Henseleit à 32°C oxygénée par un courant carbogène (95% $O_2$ et 5% $CO_2$), et on la connecte à un transducteur isométrique sous une tension basale de 0,5 g. On induit une contraction du tissu par l'addition de 0,5 µM de carbachol, on attend que la contraction se stabilise (15 min), puis on expose la préparation à la sérotonine (1 µM) afin de quantifier la relaxation maximale. On lave le tissu et, après de 20 min, on ajoute à nouveau 0,5 µM de carbachol, et on expose la préparation au composé à étudier, en concentrations cumulées croissantes de 0,1 à 1 µM.

Les composés qui induisent une relaxation sont considérés comme des agonistes 5-$HT_4$.

Pour les composés qui n'induisent pas de relaxation, la préparation est exposée à la sérotonine en concentrations cumulées croissantes, de 0,1 nM jusqu'à une concentration induisant une relaxation maximale, et la courbe de relaxation due à la sérotonine, en présence du composé à étudier, est alors comparée à une courbe témoin établie en l'absence dudit composé. Si sa présence induit un déplacement de la courbe vers la droite, le composé étudié est alors considéré comme un antagoniste 5-$HT_4$.

Les résultats de ces deux essais biologiques montrent que les composés de l'invention sont de puissants ligands des récepteurs sérotoninergiques de type 5-$HT_4$, et qu'ils agissent sur ces récepteurs soit comme des agonistes, soit comme des antagonistes.

Les composés peuvent donc être utilisés pour le traitement et la prévention des désordres dans lesquels les récepteurs 5-$HT_4$ sont impliqués, que ce soit au niveau du système nerveux central, du système gastro-intestinal, du système cardio-vasculaire ou du système urinaire.

Au niveau du système nerveux central, ces désordres et troubles comprennent notamment les troubles neurologiques et psychiatriques tels que les troubles cognitifs, les psychoses, les comportements compulsifs et obsessionnels et les états de dépression et d'anxiété. Les troubles cognitifs comprennent, par exemple, les déficits de mémoire et d'attention, les états de démence (démences séniles du type de la maladie d'Alzheimer ou démences liées à l'âge), les déficiences cérébrales vasculaires, la maladie de Parkinson. Les psychoses comprennent, par exemple, la paranoïa, la schizophrénie, la manie et l'autisme. Les comportements compulsifs et obsessionnels comprennent, par exemple, les troubles alimentaires du type de la boulimie ou de la perte d'appétit. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool, de drogue, la manie, les désordres affectifs saisonniers, les migraines, les nausées.

Au niveau du système gastro-intestinal, ces désordres et troubles comprennent notamment les vomissements induits par un traitement médical, les troubles directs ou indirects de la gastromotilité de l'oesophage, de l'estomac ou des intestins, les maladies spécifiques comme la dyspepsie, l'ulcère, le reflux gastro-oesophagien, la flatulence, le syndrome du côlon irritable, les troubles de la sécrétion intestinale, les diarrhées, par exemple celles induites par le choléra ou par le syndrome carcinoïde.

Au niveau du système cardio-vasculaire, ces désordres et troubles comprennent notamment les pathologies liées, directement ou indirectement, aux arythmies cardiaques.

Au niveau du système urinaire, ces désordres et troubles comprennent notamment les incontinences de toutes sortes, ainsi que leurs causes ou conséquences, par exemples les infections, les calculs ou les dommages rénaux.

Les composés de l'invention peuvent être présentés sous toutes formes de compositions appropriées à l'administration entérale ou parentérale, telles que comprimés, dragées, gélules, capsules, suspensions ou solutions buvables ou injectables telles que sirops ou ampoules, timbres transdermiques, suppositoires, etc, associés à des excipients convenables, et dosés pour permettre une administration journalière de 0,001 à 20 mg/kg.

**Revendications**

1. Composé, éventuellement sous forme d'isomère optique pur ou de mélange de tels isomères, répondant à la formule générale (I)

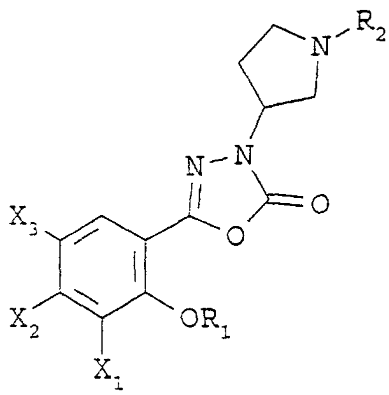

(I)

dans laquelle
$R_1$ représente un atome d'hydrogène ou un groupe $(C_1-C_4)$alkyle ou $(C_3-C_7)$ cycloalkylméthyle,
$X_1$ représente un atome d'hydrogène ou d'halogène ou un groupe $(C_1-C_4)$alcoxy ou bien
$OR_1$ et $X_1$ représentent ensemble un groupe de formule $-OCH_2O-$, $-O(CH_2)_2-$, $-O(CH_2)_3-$, $-O(CH_2)_2O-$ ou $-O(CH_2)_3O-$,
$X_2$ représente un atome d'hydrogène, un groupe amino ou un groupe de formule générale $-NHCO_2R$ dans laquelle R représente un groupe $(C_1-C_4)$alkyle ou phényl $(C_1-C_2)$ alkyle,
$X_3$ représente un atome d'hydrogène ou d'halogène, et
$R_2$ représente un atome d'hydrogène ou un groupe $(C_1-C_6)$alkyle, phényl$(C_1-C_4)$alkyle ou [(4-diméthylamino)pipéridin-1-ylcarbonyl]$(C_2-C_4)$alkyle,
à l'état de base ou de sel d'addition à un acide.

2. Procédé de préparation de composés selon la revendication 1, **caractérisé en ce qu'**on fait réagir un ester de formule générale (II)

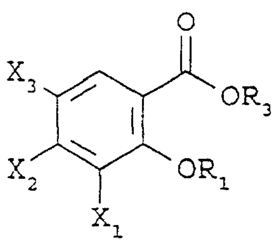

(II)

dans laquelle $R_1$, $X_1$, $X_2$ et $X_3$ sont tels que définis dans la revendication 1 et $R_3$ représente un groupe méthyle ou éthyle, avec l'hydrate d'hydrazine, pour obtenir un hydrazide de formule générale (III)

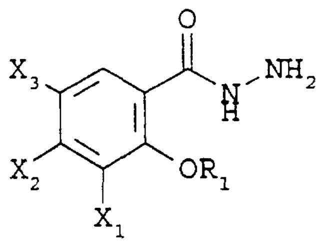

(III)

dont on obtient la cyclisation en oxadiazole de formule générale (IV)

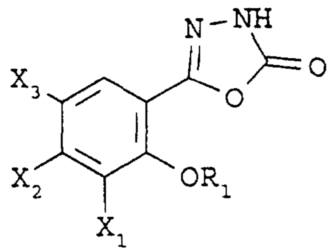

(IV)

soit au moyen de phosgène, dans un solvant aprotique, soit au moyen de chloroformiate de phényle, dans un solvant aprotique, puis on fait réagir l'oxadiazole de formule générale (IV) avec un alcool de formule générale (V)

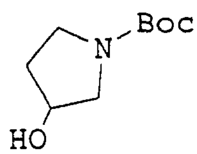

dans laquelle Boc représente un groupe (1,1-diméthyléthoxy)-carbonyle, en présence de triphénylphosphine et d'azodicarboxylate d'éthyle, dans un solvant aprotique, pour obtenir un composé de formule générale (I) dans laquelle $R_2$ représente un atome d'hydrogène, et finalement on soumet ce dernier à une alkylation au moyen d'un dérivé de formule générale $R_2$-X, dans laquelle X représente un groupe partant, et $R_2$ est tel que défini dans la revendication 1, mais différent d'un atome d'hydrogène.

**3.** Médicament **caractérisé en ce qu'**il consiste en un composé selon la revendication 1.

**4.** Composition pharmaceutique **caractérisée en ce qu'**elle contient un composé selon la revendication 1, associé à un excipient.

**Patentansprüche**

**1.** Gegebenenfalls in Form des reinen optischen Isomeren oder einer Mischung solcher Isomeren vorliegende Verbindung der allgemeinen Formel (I):

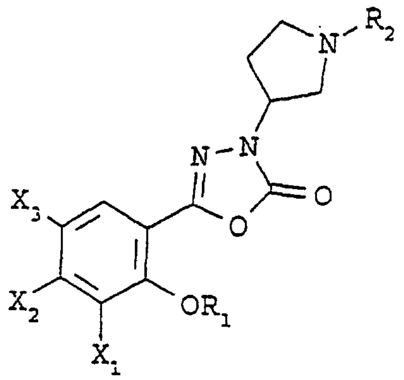

(I)

in der
$R_1$ ein Wasserstoffatom oder eine $(C_1-C_4)$-Alkylgruppe oder $(C_3-C_7)$-Cycloalkylmethylgruppe,
$X_1$ ein Wasserstoffatom, ein Halogenatom oder eine $(C_1-C_4)$-Alkoxygruppe oder
$OR_1$ und $X_1$ gemeinsam eine Gruppe der Formel $-OCH_2O-$, $-O(CH_2)_2-$, $-O(CH_2)_3-$, $-O(CH_2)_2O-$ oder $-O(CH_2)_3O-$,
$X_2$ ein Wasserstoffatom, eine Aminogruppe oder eine Gruppe der allgemeinen Formel $-NHCO_2R$, in der R eine $(C_1-C_4)$-Alkylgruppe oder eine Phenyl-$(C_1-C_2)$-alkylgruppe darstellt,
$X_3$ ein Wasserstoffatom oder ein Halogenatom und
$R_2$ ein Wasserstoffatom oder eine $(C_1-C_4)$-Alkylgruppe, Phenyl-$(C_1-C_4)$-alkylgruppe oder [(4-Dimethylamino)-piperidin-1-ylcarbonyl]-$(C_2-C_4)$-alkylgruppe bedeuten, in Form der Base oder eines Säureadditionssalzes.

**2.** Verfahren zur Herstellung der Verbindungen nach Anspruch 1, **dadurch gekennzeichnet, daß** man einen Ester der allgemeinen Formel (II)

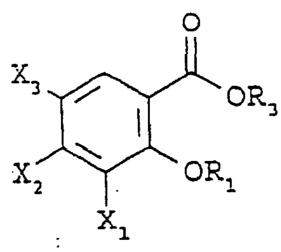

(II)

in der $R_1$, $X_1$, $X_2$ und $X_3$ die in Anspruch 1 angegebenen Bedeutungen besitzen und $R_3$ eine Methyl- oder Ethyl-Gruppe darstellt, mit Hydrazinhydrat umsetzt zur Bildung eines Hydrazids der allgemeinen Formel (III):

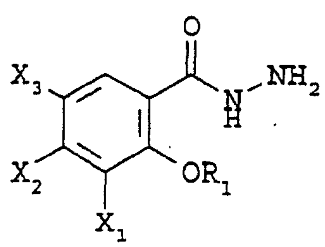

(III).

aus dem man entweder mit Hilfe von Phosgen in einem aprotischen Lösungsmittel, oder mit Chlorameisensäurephenylester in einem aprotischen Lösungsmittel, durch Cyclisierung das Oxadiazol der allgemeinen Formel (IV) erhält:

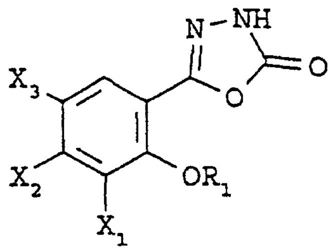

(IV)

worauf man das Oxadiazol der allgemeinen Formel (IV) in Gegenwart von Triphenylphosphin und Azodicarbonsäureethylester in einem aprotischen Lösungsmittel mit einem Alkohol der allgemeinen Formel (V):

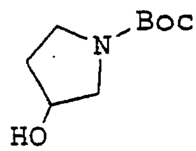

in der Boc eine (1,1-Dimethylethoxy)-carbonylgruppe darstellt, umsetzt, zur Bildung einer Verbindung der allgemeinen Formel (I), in der $R_2$ ein Wasserstoffatom bedeutet, die man schließlich einer Alkylierung mit einem Derivat der allgemeinen Formel $R_2$-X, in der X eine austretende Gruppe darstellt und $R_2$ die in Anspruch 1 angegebenen Bedeutungen besitzt, jedoch von einem Wasserstoffatom verschieden ist, unterwirft.

3. Arzneimittel, **dadurch gekennzeichnet, daß** es aus einer Verbindung nach Anspruch 1 besteht.

4. Pharmazeutische Zubereitung, **dadurch gekennzeichnet, daß** sie eine Verbindung nach Anspruch 1 in Kombination mit einem Trägermaterial enthält.

**Claims**

1. Compound, optionally in the form of a pure optical isomer or a mixture of such isomers, corresponding to the general formula (I)

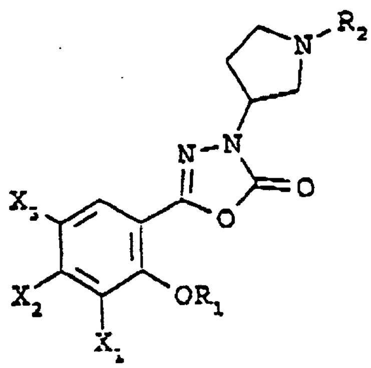

(I)

in which

| | |
|---|---|
| $R_1$ | represents a hydrogen atom or a $(C_1-C_4)$alkyl or cyclo$(C_3-C_7)$alkylmethyl group, |
| $X_1$ | represents a hydrogen or halogen atom or a $(C_1-C_4)$alkoxy group, or alternatively |
| $OR_1$ and $X_1$ | together represent a group of formula $-OCH_2O-$, $-O(CH_2)_2-$, $-O(CH_2)_3-$, $-O(CH_2)_2O-$ or $-O(CH_2)_3O-$, |
| $X_2$ | represents a hydrogen atom, an amino group or a group of general formula $-NHCO_2R$ in which R represents a $(C_1-C_4)$alkyl or phenyl$(C_1-C_2)$alkyl group, |

$X_3$ represents a hydrogen or halogen atom, and

$R_2$ represents a hydrogen atom or a $(C_1-C_6)$alkyl, phenyl$(C_1-C_4)$alkyl or [(4-dimethylamino)-piperid-1-ylcarbonyl]$(C_2-C_4)$alkyl group, in the form of a base or an addition salt with an acid.

**2.** Process for the preparation of compounds according to Claim 1, **characterized in that** an ester of general formula (II)

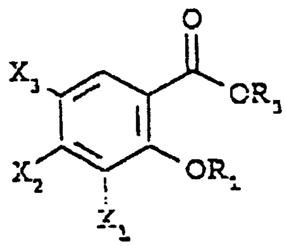

(II)

in which $R_1$, $X_1$, $X_2$ and $X_3$ are as defined in Claim 1 and $R_3$ represents a methyl or ethyl group, is reacted with hydrazine hydrate, in order to obtain a hydrazide of general formula (III)

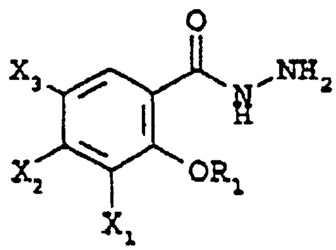

(III)

which is cyclized into an oxadiazole of general formula (IV)

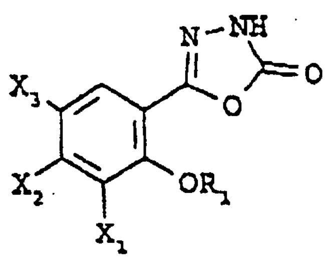

(IV)

either using phosgene, in an aprotic solvent, or using phenyl chloroformate, in an aprotic solvent, after which the oxadiazole of general formula (IV) is reacted with an alcohol of general formula (V)

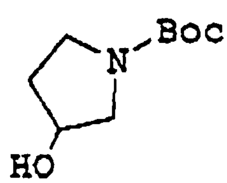

in which Boc represents a (1,1-dimethylethoxy)-carbonyl group, in the presence of triphenylphosphine and ethyl azodicarboxylate, in an aprotic solvent, in order to obtain a compound of general formula (I) in which $R_2$ represents a hydrogen atom, and, finally, the latter compound is subjected to an alkylation using a derivative of general formula $R_2$-X, in which X represents a leaving group, and $R_2$ is as defined in Claim 1, but is other than a hydrogen atom.

3. Medicinal product, **characterized in that** it consists of a compound according to Claim 1.

4. Pharmaceutical composition, **characterized in that** it contains a compound according to Claim 1, combined with an excipient.